# EUROPEAN PATENT SPECIFICATION

(11) **EP 2 060 594 B1**
(45) Date of publication and mention of the grant of the patent: **09.03.2011**
(21) Application number: 08019258.6
(22) Date of filing: 04.11.2008
(51) Int. Cl.: C08G 63/82, C12P 7/62

(54) **Method for producing polymer**
Verfahren zur Polymerherstellung
Procédé de production de polymère

(30) Priority: 14.11.2007 JP 2007295145; 23.04.2008 JP 2008113203
(43) Date of publication of application: 20.05.2009
(73) Proprietor: National University Corporation Hokkaido University, Sapporo-shi, Hokkaido 060-0808 (JP); AgriBioIndustry Inc., Sapporp-shi Hokkaido 063-0062 (JP)
(72) Inventor: Tajima, Kenji c/o Graduate School of Engineering, Sapporo-shi, Hokkaido 060-8628 (JP); Satou, Yasuharu c/o Graduate School of Engineering, Sapporo-shi, Hokkaido 060-8628 (JP); Munekata, Masanobu c/o Graduate School of Engineering, Sapporo-shi, Hokkaido 060-8628 (JP); Matsushima, Tokuo, Sapporo-shi, Hokkaido 063-0062 (JP); Taguchi, Seiichi, c/o Graduate School of Engineering,, Sapporo-shi, Hokkaido 060-8628 (JP)
(74) Representative: Oser, Andreas

(56) References cited:
- EP-A- 1 591 531
- WO-A-2006/126796
- US-A- 6 117 658
- US-B1- 6 225 438
- JOSSEK RALF ET AL: "In vitro synthesis of poly(3-hydroxybutyric acid) by using an enzymatic coenzyme A recycling system" FEMS MICROBIOLOGY LETTERS, vol. 168, no. 2, 15 November 1998 (1998-11-15), pages 319-324, XP002530843 ISSN: 0378-1097
- SATOH YASUHARU ET AL: "Enzyme-catalyzed poly(3-hydroxybutyrate) synthesis from acetate with CoA recycling and NADPH regeneration in vitro." JOURNAL OF BIOSCIENCE AND BIOENGINEERING, vol. 95, no. 4, 2003, pages 335-341, XP002530844 ISSN: 1389-1723
- STEINBUECHEL A ET AL: "DIVERSITY OF BACTERIAL POLYHYDROXYALKANOIC ACIDS" FEMS MICROBIOLOGY LETTERS, BLACKWELL PUBLISHING, AMSTERDAM, NL, vol. 128, no. 3, 15 May 1995 (1995-05-15), pages 219-228, XP000828495 ISSN: 0378-1097
- OUYANG T ET AL: "A New Chemical Method for Synthesizing and Recycling Acyl Coenzyme A Thioesters" JOURNAL OF ORGANIC CHEMISTRY, AMERICAN CHEMICAL SOCIETY, EASTON.; US, vol. 56, no. 11, 1 January 1991 (1991-01-01), pages 3752-3755, XP002979703 ISSN: 0022-3263

## Description

### TECHNICAL FIELD

The present invention generally relates to a method for producing a polyester polymer comprising units selected from serine and/or hydroxyalkanoate(s), and more particularly to a method for producing a biodegradable polyester polymer comprising units selected from serine and/or hydroxyalkanoate(s) for industrial application via a monomer-producing process by producing acetyl-coenzyme A (acetyl-CoA) using a acetate as a low-cost and starting substance, rather than a purified enzyme or expensive ATP.

### BACKGROUND ART

Among some polymers, chemical synthetic plastics derived from fossil fuels (e.g. petroleum), are unable to be degraded in natural environment and accumulate semipermanently in natural environment, resulting in various environmental problems. Under the circumstances, much attention has been focused on biodegradable plastics that are degraded by naturally-existing microorganisms (known as an eco-friendly polymeric material), and such material is increasingly developed so as to be provided with excellent properties towards practical use. In addition, the biodegradable plastics are expected to become leading biomaterials in biological and medical fields.

It has been conventionally known that biodegradable polyesters, which are produced and accumulated in many types of microorganisms from reproducible biological organic resources (biomass) such as sugar and vegetable oil. Among other things, polyhydroxyalkanoate (PHA) is expected to be an useful polyester due to thermoplasticity as the chemical synthetic plastics, excellent biodegradable and biocompatible properties, in which 90 or more types of monomer structures have been found (see FEMS Microbiol. Lett., 1995, 128, pp. 219-228).

PHA is produced in a microorganism using fermentation thereby (in vivo synthetic method), or outside a microorganism using a purified PHA synthase (PHA synthetic enzyme) and a PHA's monomer compound (in vitro synthetic method). Currently, PHA is usually produced according to the in vivo synthetic method, but unfortunately the microbial fermentation technique can provide a limited production volume of PHA that merely accumulates in the microorganism and a high-cost process for pulverizing microorganisms to extract and purify PHA therefrom. In addition, the in vivo synthetic method fails to assuredly produce PHA having desired properties, and providing limited types of PHA synthesized due to complex microbial metabolic pathways. Some fermentation control methods may produce copolymers, rather than intended homopolymers, and even resulting copolymers could be nonuniform in desired molar ratio (see FEMS Microbiol. Rev., 1992, 103, pp. 207-214).

Currently, to solve the problems mentioned above, PHA production is increased by promoting PHA synthase expression or the composition of copolymerized PHA is controlled by converting substrate specificity {see Japanese Unexamined Patent Application Publications No. 1995-265065 and No. 1998-108682, and Japanese Unexamined Patent Application Publication (Translation of PCT Application) No. 2001-516574T}.

Meanwhile, the in vitro synthetic method was developed as mass-preparation of PHA synthase was achieved using recombinant DNA techniques (see Proc. Natl. Acad. Sci., 1995, 92, pp. 6279-6283), and thus it produces PHA by using a purified PHA synthase and monomer as a substrate as shown above. Accordingly, a monomer can be chemically prepared to extend PHA monomer structure and PHA production volume can be controlled with a high precision, thereby solving the problems in the in vivo synthetic method. The use of the in vitro synthetic method may produce PHA having various physical and functional properties that the in vivo synthetic method cannot obtain.

However, PHA production according to the in vitro synthetic method involves using an extremely expensive hydroxyacyl CoA (HA-CoA) as a substrate monomer, which must be continuously supplied. More disadvantageously, HA-CoA synthesis with an expensive CoA is significantly complex.

To overcome these problems, CoA that is released in the reaction system as PHA polymerization proceeds is reused, and HA-CoA is continuously supplied, with reference to, e.g., a document (FEMS Microbiology Letters, 1998, 168, pp. 319-324) disclosing a method for reusing CoA by forming an acetyl-CoA from CoA that is released with an acetate, an acetyl-CoA synthetase and ATP mixed together, and by forming 3-hydroxybutyl CoA with a propionyl-CoA transferase and a 3-hydroxybutyrate mixed together, and by forming poly-3-hydroxybutyrate from which 3-hydroxybutyryl CoA is polymerized (see FEMS Microbiology Letters, 1998, 168, pp. 319-324).

The document in WO2004065609 discloses a method for continuously supplying HA-CoA by reproducing CoA in PHA production process from thioester as a starting substance. More specifically, the thioester as a starting substance is reacted with CoA to form HA-CoA in a thioester exchange reaction, resulting in PHA production via polymerization by PHA synthase. In this process, CoA, which is released during this polymerization reaction, will be reproduced in an ester exchange reaction to continuously produce and reproduce HA-CoA.

Y. Satoh et al., Journal of Bioscience and Bioengineering, Vol. 95, No. 4, 335-341 (2003), describe the enzyme-catalyzed poly(3-hydroxybutyrate) synthesis from acetate with CoA recycling and NADPH regeneration *in vitro.* The poly(3-HB) obtained by this synthesis had a weight-average molecular weight of 6.64x10⁶ and a polydispersity of 1.36.

A. Steinbüchel and H.E. Valentin, FEMS Microbiology Letters 128, 219-228 (1995), provide an overview on the diversity of biosynthetic polyhydroxyalkanoic acids.

WO 2006/126796 A1 relate to cells or plants having a producing ability of polylactate or its copolymers, and a method for preparing polylactate or its copolymers using such cells or plants.

Further, US 6,117,658 A relates to methods of making polyhydoxyalkanoates (PHA) comprising 4-hydroxybutyrate (4-HB) monomer units. The obtained PHA comprising 4-HB monomer units is said to have a polydispersity that is generally less than about 2, typically less than about 1.6, preferably less than about 1.5, and further preferably less than about 1.4 or 1.3.

Further, US 6,225,438 B1 discloses medium chain length PHA copolymer and a process for producing the same, wherein the obtained PHA can adopt various Mₙ and M_{w} values (Table 2).

Finally, T. Ouyang and D.R. Walt, Journal of Organic Chemistry, Vol. 56, No. 11, 3752-3755 (1991), describe a chemical method for synthesizing acyl coenzyme A thioesters and for recycling acetyl CoA thioesters in enzyme-catalyzed reactions. The acylating reagent is S-acylthiocholine to produce acyl-CoA.

### DISCLOSURE OF THE INVENTION

However, the method for producing PHA as disclosed in the document (FEMS Microbiology Letters, 1998, 168, pp. 319-324) involves using 3 types of enzymes that are difficult to be purified and extremely expensive ATP, leading to unachievable industrial application.

On the other hand, the invention disclosed in WO2004065609 produces a thiophenyl ester as a starting substance by thiophenyl-esterifying hydroxyalkanoate (HA). In reality, this thiophenyl esterification reaction cannot achieve CoA reproduction on industrial level, because this reaction requires a long-term process for the protection of HA's hydroxyl group and deprotection after the reaction.

In addition, according to the invention disclosed in WO2004065609, the thioester exchange reaction releases thiophenol as a remarkably toxic substance when acyl-CoA is formed from thiophenyl ester. Unfortunately, as polymerization reaction proceeds, several percents of thiophenol dissolve in an aqueous phase solution to inhibit the activity of PHA synthase.

It is, therefore, one object of the present invention to provide a method for producing a polyester polymer comprising units selected from serine and/or hydroxyalkanoate(s) of high production efficiency, productivity and thus industrial application, and a polymer composed of desired monomer units continuously producing and reproducing acetyl-CoA without using a purified enzyme or expensive ATP.

A method for producing a polyester polymer comprising units selected from serine and/or hydroxyalkanoate(s) according to the present invention comprises the following reaction, that is :
(a) a chemical thioester exchange reaction, wherein an acetyl-thioester is reacted with CoA for forming acetyl-CoA;
(b) a monomer-producing reaction, wherein at least one monomer precursor compound is reacted with the acetyl-CoA for forming a (monomer precursor)-CoA derivative;
(c) a polymerization reaction which the (monomer precursor)-CoA derivative is polymerized for forming the polyester polymer comprising units of the monomer selected from serine and/or hydroxyalkanoate(s).

The monomer precursor compound is a carboxylic acid and preferably a hydroxy acid.

Also, in this invention, polyester comprises units selected from serine or hydroxyalkanoates, preferably lactate (LA), 3-hydroxypropionate (3HP), 3-hydroxybutyrate (3HB), or 4-hydroxybutyrate (4HB), alone or in combination with each other or with other units.

In this invention, the polyester, preferably polylactate (PLA), poly-3-hydroxypropionate {P(3HP)}, poly-3-hydroxybutyrate {P(3HB)}, poly-4-hydroxybutyrate {P(4HB)}, LA-3HP-copolyester {P(LA-*co-*3HP)}, LA-3HB-copolyester {P(LA-*co-*3HB)}, LA-4HB-copolyester {P(LA-*co-*4HB)}, 3HP-3HB-copolyester {P(3HP-*co-*3HB)}, 3HP-4HB-copolyester {P(3HP-*co-*4HB)}, or 3HB-4HB-copolyester {P(3HB-*co-*4HB)} is produced as said polymer.

In this invention, the lactate (LA) is preferably Dextro-rotatory-lactate (D-lactate).

Moreover, in this invention, the chemical thioester exchange reaction preferably comprises forming acetyl-CoA from CoA, which is released from the (monomer precursor)-CoA derivative in the polymerization reaction, and the acetyl-thioester.

Also, it is preferable that in this invention, the acetyl-thioester of the chemical thigoester exchange reaction is prepared from an acetate and a thiol compound by a thioesterification reaction.

In this invention, the thioesterification reaction comprises forming acetyl-thioester from acetate, which is released in the monomer-producing reaction, and thiol compounds.

Also in this invention, the thiol compound is preferably ethylthioglycolate (ETG).

Moreover, in this invention, an enzyme used for the monomer-producing reaction is one using acetyl-CoA as a substrate.

In this invention, it is preferable that the chemical thioester exchange reaction, the monomer-producing reaction and the polymerization reaction proceed concurrently in an one pot reaction system, the one pot reaction system preferably comprising an organic solvent phase and an aqueous solvent phase.

Also in this invention, the organic solvent phase comprises the acetyl-thioester, and the aqueous phase comprises CoA, the monomer precursor compound and two enzymes catalyzing the monomer-producing reaction (b) and the polymerization reaction (c), respectively.

It is desirable that in this invention, a ratio of the concentration of the acetyl-thioester and the concentration of the monomer precursor compound is 1:1 to 10:1.

Moreover, using the method of the present invention provides for a targeted setting of the molecular weight distribution (Mw/Mn, wherein Mw denotes the weight-average molecular weight and Mn denotes the number-average molecular weight) of the polyester polymer comprising units selected from serine and/or hydroxyalkanoate(s). The particular advantage of the present invention is that a polyester polymer comprising units selected from serine and/or hydroxyalkanoate(s) and having a narrow molecular weight distribution, preferably between 1 and 3, and more preferably between 1 and 2.5 is prepared.

Accordingly, it is, of course, that this invention can produce a polymer of a favorable industrial application in a high-yield, immediate and easy manner by continuously producing and reproducing acetyl-CoA without using a purified enzyme or expensive ATP. Also, this invention can produce a polymer with a desired polymer composition due to wider choices on monomer units.

### BRIEF DESCRIPTION OF THE DRAWINGS

The above and other objects of the invention will be seen by reference to the description taken in connection with the drawings, in which:
Figure 1 is a diagram showing the reaction pathway for producing a polymer after forming acetyl-CoA from acetyl-thioester and forming (monomer precursor)-CoA derivative;
Figure 2 is a diagram showing the reaction pathway for producing a polymer after forming acetyl-CoA from acetyl-thioester and forming (monomer precursor)-CoA derivative in the one pot reaction system;
Figure 3 is a diagram showing the thioesterification reaction for forming acetyl-thioester from acetate;
Figure 4 is a diagram showing the reaction pathway for producing P (3HB) after forming acetyl-CoA from acetyl-ETG and forming (monomer precursor)-CoA derivative in the one pot reaction system;
Figure 5 is a diagram showing the reaction pathway for producing P (3HB-*co-*LA) after forming acetyl-CoA from acetyl-ETG and forming (monomer precursor)-CoA derivative in the one pot reaction system;
Figure 6 is a diagram showing the reaction (1) for forming acetyl-TP from acetate in Example 1 and the reaction (2) for forming acetyl-ETG from acetate in Example 1;
Figure 7 is a diagram showing the reaction pathway (1) for producing P (3HB) after forming acetyl-CoA from acetyl-TP and forming (*R*)-3HB-CoA and the reaction pathway (2) for producing P (3HB) after forming acetyl-CoA from acetyl-ETG and forming (*R*)-3HB-CoA ("n" in Figure 7 is an integer 1 or more);
Figure 8 is a graph showing the results of NMR measured for compounds obtained using acetyl-ETG with the same concentration as monomer precursor compound in Example 1;
Figure 9 is a graph showing the results of NMR measured for compounds obtained using acetyl-ETG with the concentration 10 times that of monomer precursor compound in Example 1;
Figure 10 is a graph showing the results of synthetic reaction rate and synthetic amount measured in P (3HB) according to the type of acetyl-thioester in Example 2;
Figure 11 is a graph showing the results of reaction rate and amount measured in P (3HB) production according to difference in the ratio of the volume of the organic solvent phase comprising acetyl-ETG and the amount of the aqueous phase comprising (*R*)-3HB in Example 3;
Figure 12 is a diagram showing the reaction pathway for producing P (3HB-*co-*3HP) after forming acetyl-CoA from acetyl-ETG and forming (*R*)-3HB-CoA or 3HP-CoA in Example 4 ("x" and "y" in Figure 12 are integers 1 or more); and
Figure 13 is a graph showing the results of NMR measured for compounds obtained in Example 4.
Figure 14 is a diagram showing the reaction pathway for producing P (3HB-*co*-LA) after forming acetyl-CoA from acetyl-ETG and forming (*R*)-3HB-CoA or LA-CoA in Example 5 ("x" and "y" in Figure 14 are integers 1 or more); and
Figure 15 is a graph showing the results of NMR measured for compounds obtained in Example 5.

### BEST MODE FOR CARRYING OUT THE INVENTION

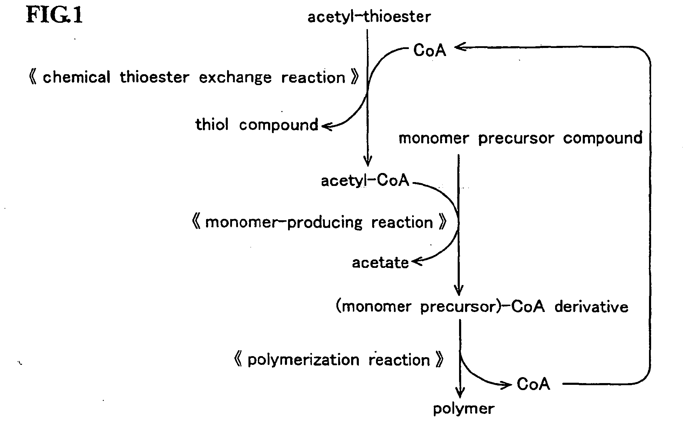

A preferred embodiment of the method for producing a polymer according to the present invention will be described with reference to Figures 1 to 3. As shown in Figure 1, the method for producing a polymer of this embodiment comprises a chemical thioester exchange reaction, a monomer-producing reaction and a polymerization reaction.

The chemical thioester exchange reaction is a chemical reaction that reacts an acetyl-thioester as an acetyl donor with CoA to form acetyl-CoA and releases thiol compounds. The monomer-producing reaction reacts the acetyl-CoA produced in the chemical thioester exchange reaction with at least one monomer precursor compound, forms a (monomer precursor)-CoA derivative by substituting CoA for a hydroxyl group of the monomer precursor compound and releases acetate. The polymerization reaction forms a polymer by polymerizing the (monomer precursor)-CoA derivative obtained by the monomer-producing reaction and releases CoA.

A monomer precursor compound in the monomer-producing reaction may be carboxylic acid, preferably hydroxy acid or unsaturated fatty acid. The hydroxy acid or unsaturated fatty acid is not particularly limited if it forms a (monomer precursor)-CoA derivative by the monomer-producing reaction and then produces a polymer by the polymerization reaction after, but may be aliphatic hydroxy acid such as tartrate, glycerate, acrylate, crotonate, aminocrotonate, hydroxycrotonate, pentenate, hexanoate, octanoate, malic acid, tartaric acid, citramalic acid, citric acid, isocitric acid, leucic acid, mevalonic acid, pantoic acid, ricinoleic acid, ricinelaidic acid, cerebronic acid, quinic acid, shikimic acid, serine (Ser) and HA, or aromatic hydroxy acid such as salicylic acid, hydroxymethyl benzoic acid, vanillic acid, syringic acid, pyrocatechuic acid, resorcyclic acid, protocatechuic acid, gentisic acid, orsellinic acid, gallic acid, mandelic acid, benzilic acid, atrolactic acid , melilotic acid, phloretic acid, coumaric acid, umbellic acid, caffeic acid, ferulic acid and sinapic acid, preferably Ser or HA.

Next, HA in this invention may be lactic acid (2-hydroxypropionate ; LA), glycolic acid, 3-hydroxypropionate (3HP), 3-hydroxybutyrate (3HB), 3-hydroxy valerate (3HV), 3-hydroxyhexanoate, 3-hydroxyheptanoate, 3-hydroxyoctanoate, 3-hydroxynonanoate, 3-hydroxydecanoate, 3-hydroxyundecanoate, 3-hydroxydodecanoate, 3-hydroxydodecenoate, 3-hydroxytetradecanoate, 3-hydroxyhexadecanoate, 3-hydroxyoctadecanoate, 4-hydroxybutyrate (4HB), 4-hydroxy valerate, 5-hydroxy valerate, 6-hydroxyhexanoate and hydroxylauric acid, preferably LA, 3HP, 3HB or 4HB.

There are D-enantiomer and L-enantiomer of LA. LA as a monomer precursor compound in this invention may be both of them, preferably D-enantiomer in this embodiment.

The polymers that can be produced in the method for producing a polymer according to this invention are polyesters. The polyesters in this invention may be PHA that homopolymers of the HA or copolymers, randompolymers or blockpolymers comprising the HA selected from the HA, preferably poly LA (PLA), poly (3-hydroxypropionate) {P(3HP)}, poly (3-hydroxybutyrate) {P(3HB)}, poly (4-hydroxybutyrate) {P(4HB)}, copolymer of LA and 3HP {P(LA-*co-*3HP)}, copolymer of LA and 3HB {P(LA-*co-*3HB)}, copolymer of LA and 4HB {P(LA-*co-*4HB)}, copolymer of 3HP and 3HB {P(3HP-*co-*3HB )}, copolymer of 3HP and 4HB {P(3HP-*co-*4HB)} and copolymer of 3HB and 4HB {P(3HB-*co-*4HB)}. In addition, the polyesters include units selected from Ser or HA. Therefore these units may be preferable that homopolymer of Ser (PS), copolymers, randompolymers or blockpolymers comprising Ser and HA selected from 3HP, 3HB or 4HB, alone, or in combination with each other or with other units. It is preferable that the polymers in this invention are biodegradable.

The method for producing a polymer according to the present invention can form an acetyl-CoA by reacting CoA released in the polymerization reaction with an acetyl-thioester in the chemical thioester exchange reaction. This means, CoA used in forming the acetyl-CoA in the chemical thioester exchange reaction can be reproduced from CoA released from a (monomer precursor)-CoA derivative in the polymerization reaction. Also, CoA can be supplied to form acetyl-CoA in the chemical thioester exchange reaction.

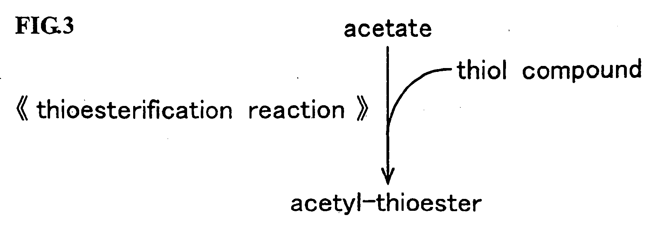

A method for producing acetyl-thioester as a starting substance in the chemical thioester exchange reaction is not particularly limited if acetyl-thioester can be formed using low-cost compounds, but preferably employs a thioesterification reaction for reacting acetate with thiol compounds, as shown in Figure 3, in view of one-step and easy process without using expensive compounds.

Meanwhile, acetate released in the monomer-producing reaction reacts with thiol compounds in the thioesterification reaction, resulting in the production of acetyl-thioester. Specifically, acetate used in forming acetyl-thioester in the thioesterification reaction can be reproduced from acetate released in the monomer-producing reaction. Also, acetate can be supplied to form acetyl-thioester in the thioesterification reaction.

Here, thiol compounds in this invention are not particularly limited if they have a thiol group that can be given to CoA to form an acetyl-CoA, but preferably aliphatic thiol and aromatic thiol, and more specifically alkylthiol and cycloalkylthiol of C1 to C18 such as ethanethiol, propanethiol, benzylmercaptan, 2-mercaptoethyl ether and cyclohexylthiol, thiol comprising hydroxyl groups such as mercaptoethanol and p-mercaptophenol, thiol comprising carboxylic acid ester such as methylmercaptoacetate and ethylmercaptopropionate, aromatic thiol such as thiophenol (TP), toluenethiol and naphthalenethiol, nitrogenous aromatic thiol such as 2-mercapto-1-methylimidazole, mercaptopyridine, mercaptothiazoline, mercaptobenzothiazoline, mercaptobenzoxazole and mercaptopyrimidine, thioglycolate such as methylthioglycolate, ethylthioglycolate (ETG), propylthioglycolate, isopropylthioglycolate, butylthioglycolate, n-amylthioglycolate, isoamylthioglycolate, hexylthioglycolate, octylthioglycolate, n-decylthioglycolate, laurylthioglycolate, tridecylthioglycolate, stearylthioglycolate, thionalide, furfurylmercaptan, cyclohexylthioglycolate and hydroxyethylthioglycolate, preferably TP or thioglycolate, more preferably thioglycolate. A preferred thiol compound in this embodiment is ETG due to an ability to form acetyl-thioester from acetate stably, maintain synthase activity for untoxic property, and high production rate and high production yield of polymer.

On the other hand, an enzyme used in the monomer-producing reaction is preferably one using acetyl-CoA as a substrate, because the monomer-producing reaction forms a (monomer precursor)-CoA derivative from acetyl-CoA and the hydroxy acid as a substrate. The enzyme having acetyl-CoA as a substrate may be CoA transferase. The CoA transferase may be acetoacetyl-CoA transferase, caffeoyl-CoA transferase, coumaroyl-CoA transferase, glutaryl-CoA transferase, crotonyl-CoA transferase, sinapoyl-CoA transferase, cinnamoyl-CoA transferase, succinyl-CoA transferase, 3-hydroxybutanoyl-CoA transferase, hydroxymethyl glutaryl-CoA transferase, feruloyl-CoA transferase, propionyl-CoA transferase (PCT) and malonyl-CoA transferase, furthermore a favorable CoA transferase can be selected according to hydroxy acid as a substrate. For example, if hydroxy acid is 3HB, 3HP, 4HB, LA or Ser, PCT can be used.

The CoA transferase-derived microorganisms are not particularly limited if they have an enzyme that can transfer a CoA group from the acetyl-CoA to the monomer precursor compound, but if the enzyme is propionyl-CoA transferase, the microorganisms may be genus Clostridium, genus Megasphaera, genus Alkaliphilus, genus Thermosinus, genus Pelotomaculum, genus Listeria, genus Ralstonia, genus Syntrophobacter, genus Fusobacterium, genus Syntrophus, genus Mycobacterium, genus Dechloromonas, genus Bacillus, genus Rhodoferax, genus Bradyrhizobium, genus Polynucleobacter, genus Eubacterium, genus Rhizobium, genus Oceanospirillum, genus Vibrio, genus Burkholderia, genus Pseudomonas, genus Shewanella and genus Escherichia, more specifically Clostridium propionicum, Clostridium kluyveri, Megaspkaera elsdenii, Clostridium novyi, Clostridium tetani, Clostridium perfringens, Clostridium beijerinckii, Alkaliphilus metalliredigens, Alkaliphilus oremlandii, Thermosinus carboxy-divorans, Pelotomaculum thermopropionicum, Listeria monocytogenes, Listeria welshimeri, Ralstonia eutropha, Syntrophobacter fumaroxidans, Fusobacterium nucleatum, Syntrophus aciditrophicus, Mycobacterium smegmatis, Dechloromonas aromatica, Bacillus halodurans, Rhodoferax ferrireducens, Bradyrhizobium japonicum, Polynucleobacter sp., Eubacterium dolichum, Rhizobium leguminosarum, Oceanospirillum sp., Vibrio shilonii, Burkholderia phyto-firmans, Pseudomonas mendocina, Shewanella sediminis and Escherichia coli.

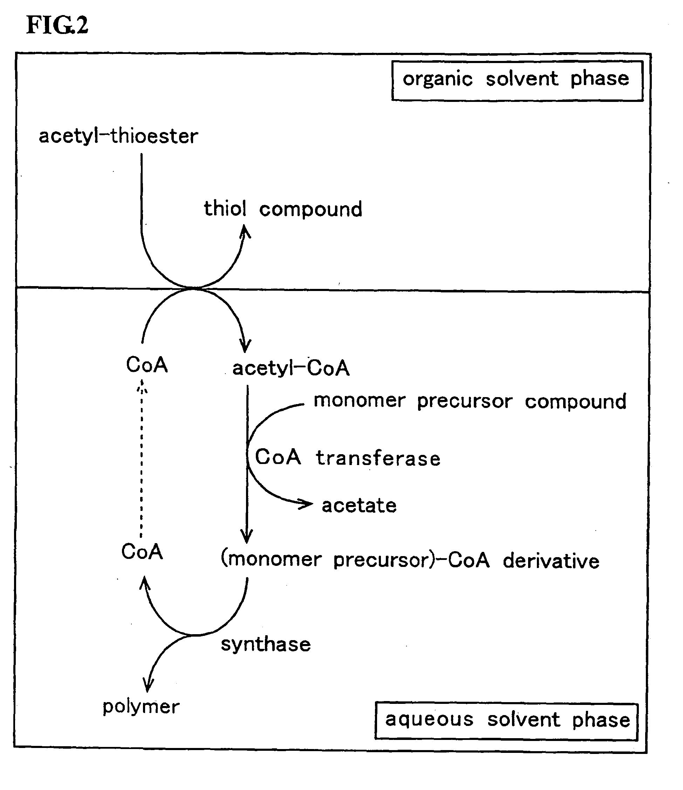

Next, the method for producing a polymer according to the present invention includes at least the chemical thioester exchange reaction, the monomer-producing reaction and the polymerization reaction, and may also include other reactions like hydration by hydratase. In addition, the chemical thioester exchange reaction, the monomer-producing reaction and the polymerization reaction can separately proceed, and as shown in Figure 2, they can concurrently proceed in one pot reaction system. The one pot reaction system can include the thioesterification reaction, and preferably comprises an organic solvent phase and an aqueous phase.

To produce a polymer in the one pot reaction system including the organic solvent phase and the aqueous phase, it is preferable that the organic solvent phase comprises acetyl-thioester, and the aqueous phase includes CoA, monomer precursor compound CoA transferase and synthase. As shown in Figure 2, the acetyl-thioester in the organic solvent phase and CoA in the aqueous phase form thiol compounds and acetyl-CoA by the chemical thioester exchange reaction at the interface therebetween, and acetyl-CoA and monomer precursor compound form acetate and (monomer precursor)-CoA derivative in the aqueous phase by the monomer-producing reaction by catalysis of an enzyme using acetyl-CoA as a substrate. Subsequently, (monomer precursor)-CoA derivatives in the aqueous phase are polymerized to produce a polymer by catalysis of an enzyme according to the polymerization reaction.

Now, the enzyme according to the polymerization reaction may be PHA synthase. PHA synthase in this invention includes enzymes that polymerize not only HA for forming PHA, but also LA for forming PHA, and HA and LA for forming copolymers, randompolymers or blockpolymers. PHA synthase is favourably selected according to the (monomer precursor)-CoA derivative as a substrate. For instance, when the (monomer precursor)-CoA derivative is 3HB-CoA, 3HP-CoA, 4HB-CoA or LA-CoA, alone or in combination with each other or with other units, PHA synthase of genus Ralstonia-derived, genus Pseudomonas-derived, or conservative-amino-acid replacement variant-derived can be used.

The PHA synthase-derived microorganisms are not particularly limited if they have an enzyme that can synthesize PHA using HA-CoA as a substrate, but the microorganisms may be genus Ralstonia, genus Burkholderia, genus Methylibium, genus Pseudomonas, genus Cupriavidus, genus Polaromonas, genus Alcaligenes, genus Azohydromonas, genus Rhodoferax, genus Acidovorax, genus Verminephrobacter, genus Polynucleobacter, genus Bordetella, genus Zoogloea, genus Herminiimonas, genus Dechloromonas, genus Azoarcus, genus Bradyrhizobium, genus Azotobacter, genus Oceanospirillum, genus Chromobacterium, genus Nitrococcus, genus Alkalilimnicola, genus Magnetospirillum, genus Halorhodospira, genus Rhodospirillum, genus Rubrobacter, genus Parvibaculum, genus Acidiphilium, genus Sphingomonas, genus Saccharophagus, genus Photobacterium, genus Chromohalobacter, genus Azorhizobium, genus Methylobacterium, genus Vibrio, genus Rhodopseudomonas, genus Bacillus, genus Roseiflexus, genus Syntrophomonas, genus Chloroflexus, genus Myxococcus, genus Novosphingobium, genus Haloarcula, genus Cenarchaeum, genus Synechococcus, genus Synechocystis, genus Allochromatium, genus Microscilla, genus Chlorogloeopsis, genus Ectothiorhodospira, genus Xanthomonas, genus Nitrococcus mobilis, genus Marinobacter, genus Alcanivorax, genus Hahella, genus Acinetobacter, genus Aeromonas, genus Limnobacter and genus Parvularcula, and more specifically Ralstonia eutropha, Ralstonia metallidurans, Ralstonia solanacearum, Ralstonia pickettii, Burkholderia multivorans, Burkholderia pseudomallei, Burkholderia dolosa, Burkholderia mallei, Burkholderia ambifaria, Burkholderia cenocepacia, Burkholderia thailandensis, Burkholderia phymatum, Burkholderia xenovorans, Burkholderia vietnamiensis, Methylibium petroleiphilum, Pseudomonas putida, Pseudomonas oleovorans, Cupriavidus necator, Polaromonas naphthalenivorans, Alcaligenes sp., Azohydromonas lata, Rhodoferax ferrireducens, Acidovorax avenae, Verminephrobacter eiseniae, Polynucleobacter sp., Bordetella pertussis, Zoogloea ramigera, Bordetella bronchiseptica, Bordetella parapertussis, Bordetella avium, Herminiimonas arsenicoxydans, Limnobacter sp., Dechloromonas aromatica, Azoarcus sp., Bradyrhizobium japonicum, Azotobacter vinelandii, Oceanospirillum sp., Chromobacterium violaceum, Nitrococcus mobilis, Alkalilimnicola ehrlichei, Magnetospirillum magneticum, Halorhodospira halophila, Rhodospirillum rubrum, Magnetospirillum gryphiswaldense, Rubrobacter xylanophilus, Parvibaculum lavamentivorans, Acidiphilium cryptum, Sphingomonas sp., Saccharophagus degradans, Photobacterium profundum, Chromohalobacter salexigens, Azorhizobium caulinodans, Methylobacterium sp., Vibrio alginolyticus, Rhodopseudomonas palustris, Bacillus anthracis, Bacillus cereus, Bacillus thuringiensis, Bacillus weihenstephanensis, Bacillus megaterium, Rubrobacter xylanophilus, Roseiflexus castenholzii, Syntrophomonas wolfei, Chloroflexus aggregans, Myxococcus xanthus, Novosphingobium aromaticivorans, Haloarcula marismortui, Haloarcula hispanica, Halorhodospira halophila, Cenarchaeum symbiosum, Synechococcus sp., Synechocystis sp., Allochromatium vinosum, Microscilla marina, Chlorogloeopsis fritschii, Ectothiorhodospira shaposhnikovii, Xanthomonas campestris, Nitrococcus mobilis, Marinobacter aquaeolei, Alcanivorax borkumensis, Hahella chejuensis, Acinetobacter baumannii, Aeromonas salmonicida and Parvularcula bermudensis.

In the method for producing a polymer according to this invention, when the chemical thioester exchange reaction, the monomer-producing reaction, and the polymerization reaction concurrently proceed in the one pot reaction system, a ratio (mmol/L) of the molecular concentration of the acetyl-thioester in the organic solvent phase and the molecular concentration of the monomer precursor in the aqueous phase is preferably 1:1 to 10:1 in view of production rate and production yield.

The molecular weight distribution for a polymer produced in the method for producing a polymer according to this invention is not particularly limited if the polymer is produced by the above-mentioned method, but it is preferably between 1 and 3, and more preferably between 1 and 2.5 in view of the quality and repeatability in polymer production.

As stated above, the method for forming acetyl-CoA via forming acetyl-thioester from acetate in this embodiment is industrially useful due to low-cost substances such as acetate as a raw material, without using expensive materials such as ATP and enzymes requiring to be purified or carefully handled with. Additionally, a released monomer precursor compound, such as LA produced by readily produced and low-cost lactic acid fermentation, can be directly used in the monomer-producing reaction. Specifically, the method for producing a polymer according to this invention can readily produce a polymer having a desired composition by selecting a desired monomer.

Next, an embodiment of the method for producing a polymer according to the present invention will be described. In this embodiment, an acetyl-ETG is formed from acetate and ETG by a thioesterification reaction. By adding the acetyl-ETG to an organic solvent phase composed of hexane and adding CoA, HA, CoA transferase and PHA synthase to an aqueous phase, the chemical thioester exchange reaction, the monomer-producing reaction and the polymerization reaction gradually proceed to produce a polymer.

Specifically, the acetyl-ETG in a hexane phase and the CoA in an aqueous phase cause a chemical thioester exchange reaction at the interface therebetween to release ETG is released in the hexane phase and form acetyl-CoA in the aqueous phase. Subsequently, the acetyl-CoA and HA release acetate in the aqueous phase by a monomer-producing reaction by CoA transferase and form a HA-CoA derivative. Finally, the HA-CoA is polymerized by the PHA synthase in the aqueous phase to release CoA in the aqueous phase and to produce a polymer.

The CoA released in the aqueous phase in the polymerization reaction is reproduced in the chemical thioester exchange reaction, thereby continuously supplying acetyl-CoA because of CoA recycling. In addition, acetate that is released in the aqueous phase in the monomer-producing reaction can be extracted to reuse as a raw material for acetyl-ETG.

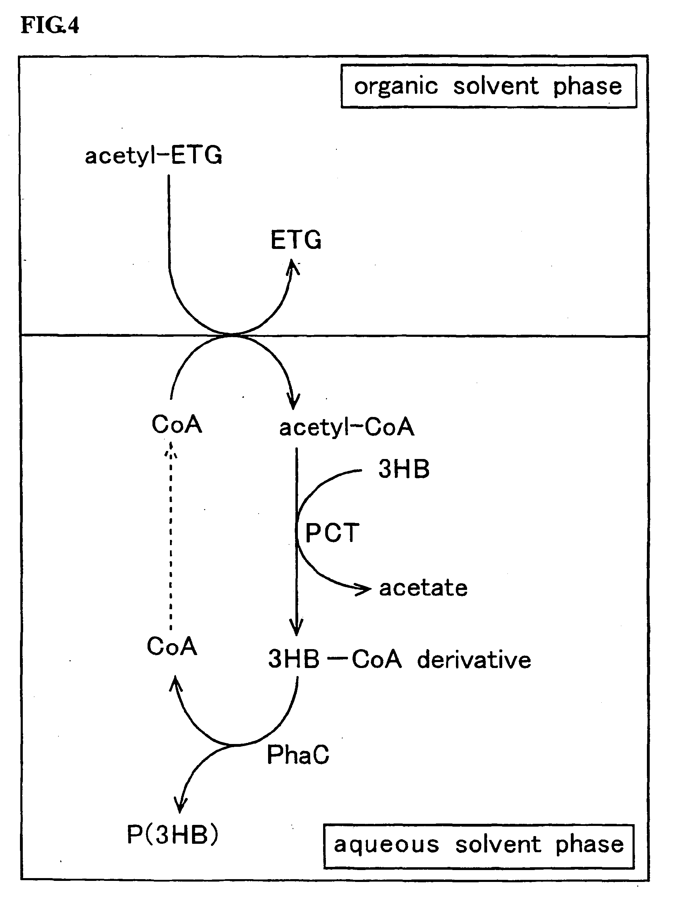

Then, in this embodiment, a method for forming P(3HB) after forming acetyl-CoA from acetate and ETG and forming 3HB-CoA derivative will be described with reference to Figure 4, Figure 6(2) and Figure 7(2).

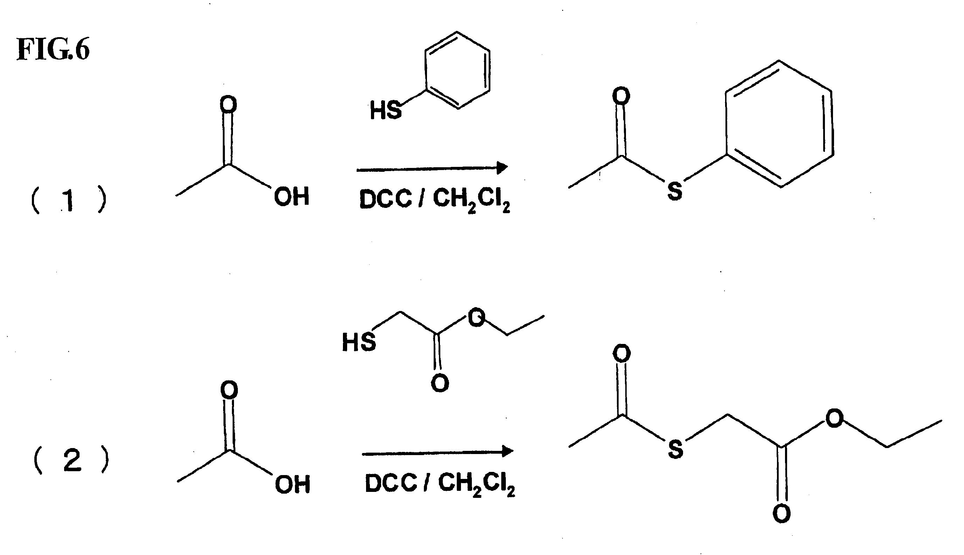

Firstly, a method for forming an acetyl-ETG from acetate in one step by a thioesterification reaction will be described. As shown in Figure 6 (2), by reacting acetate with ETG in dichloromethane (CH₂Cl₂) having dicyclohexylcarbodiimide (DCC), acetyl-ETG is formed in one step.

Next, in this embodiment, a method for producing P (3HB) from acetyl-ETG and 3HB in an one pot reaction system will be described. Specifically, as shown in Figure 4, the chemical thioester exchange reaction, the monomer-producing reaction for forming 3HB-CoA derivative from 3HB as a monomer component and the polymerization reaction for producing P(3HB) concurrently proceed in the one pot reaction system.

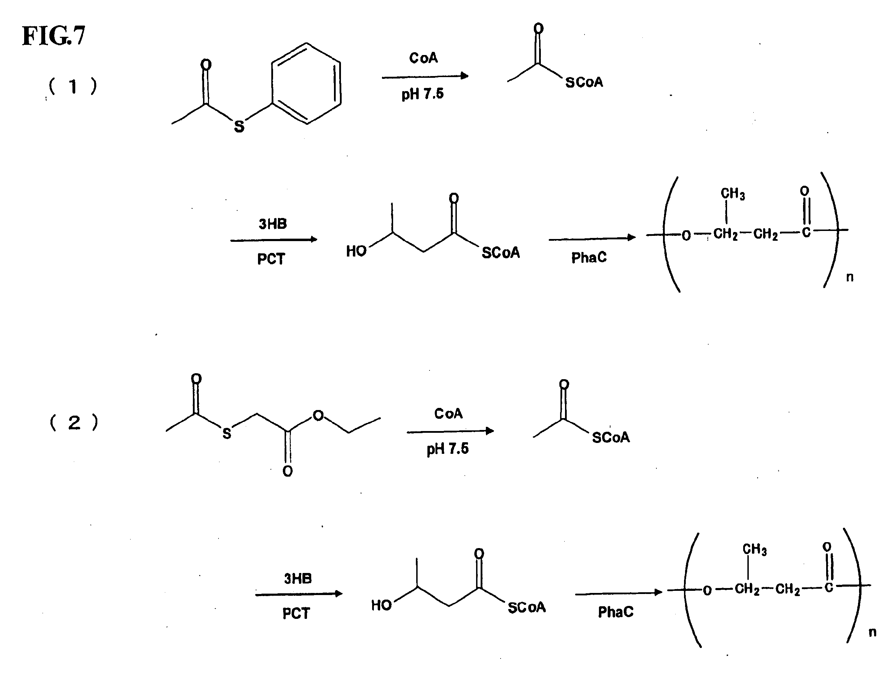

In this production method, by dissolving the acetyl-ETG in an organic solvent phase using hexane and adding CoA, 3HB, PCT and PHA synthase to a sodium hydrogenphosphate solution as an aqueous phase, a chemical thioester exchange reaction is performed at the interface between the organic solvent phase and the aqueous phase. Subsequently, by performing the monomer-producing reaction and the polymerization reaction in the aqueous phase, P(3HB) is produced. Figure 7(2) shows the reaction pathway.

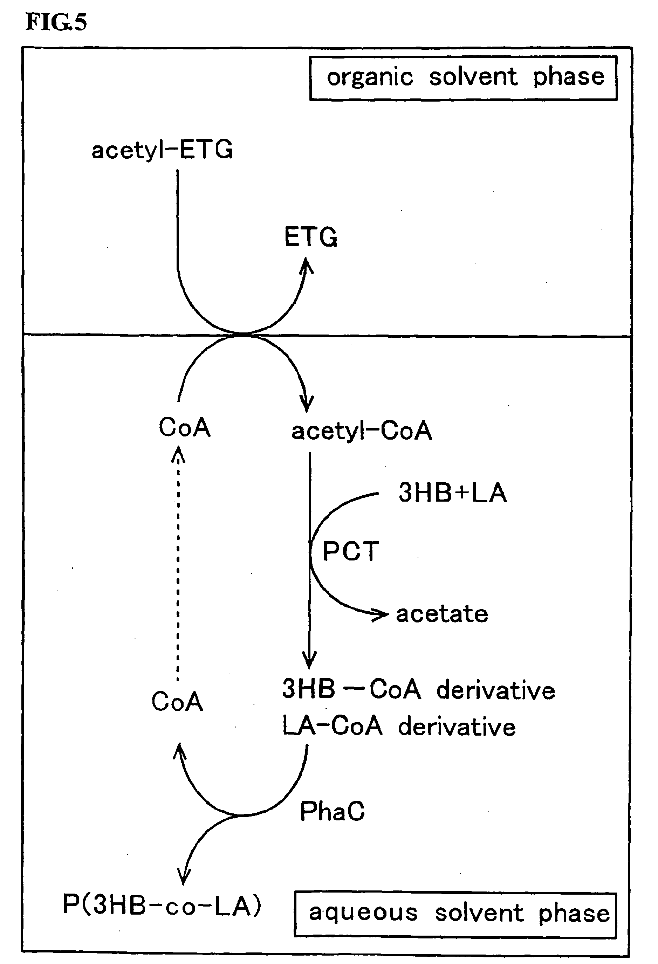

Subsequently, in this embodiment, a method for forming P(3HB-*co-*LA) after forming acetyl-CoA from acetate and ETG and forming 3HB-CoA derivative and 3LA-CoA derivative will be described with reference to Figure 5, Figure 6(2) and Figure 14.

Firstly, a method for forming an acetyl-ETG from acetate in one step by a thioesterification reaction will be described. As shown in Figure 6(2), by reacting acetate with ETG in dichloromethane (CH₂Cl₂) having dicyclohexylcarbodiimide (DCC), acetyl-ETG is formed in one step.

Next, in this embodiment, a method for producing P(3HB-*co-*LA) from acetyl-ETG, 3HB and LA in an one pot reaction system will be described. Specifically, as shown in Figure 5, the chemical thioester exchange reaction, the monomer-producing reaction for forming 3HB-CoA derivative and LA-CoA derivative from 3HB and LA respectively as monomer components and the polymerization reaction for producing P(3HB-*co-*LA) concurrently proceed in the one pot reaction system.

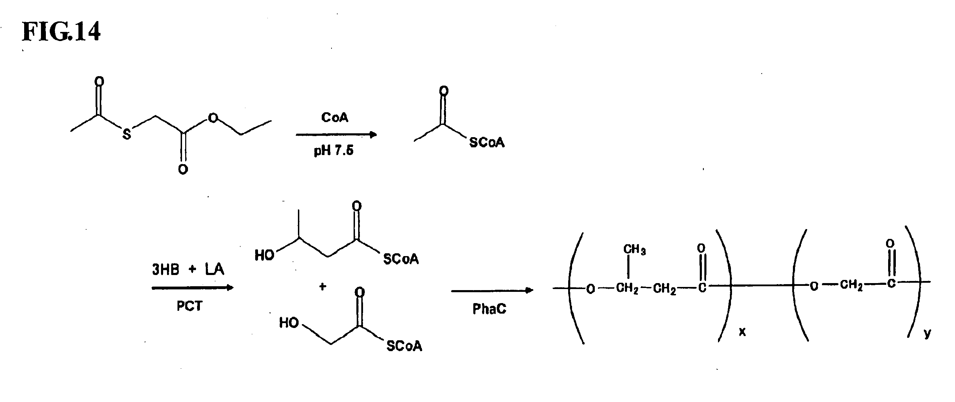

In this production method, by dissolving the acetyl-ETG in an organic solvent phase using hexane and adding CoA, 3HB, LA, PCT and PHA synthase to a sodium hydrogenphosphate solution as an aqueous phase, a chemical thioester exchange reaction is performed at the interface between the organic solvent phase and the aqueous phase. Subsequently, by performing the monomer-producing reaction and the polymerization reaction in the aqueous phase, P(3HB-*co-*LA) is produced. Figure 14 shows the reaction pathway.

In this embodiment, the ratio of the volumes in the organic solvent phase and the aqueous phase is not particularly limited if P(3HB), P(3HB-*co-*3HP) or P(3HB-*co-*LA) as an objective product can be produced from the acetyl-ETG in the organic solvent phase. However, in view of more immediate and high-yield production, a ratio (mmol/mL) of the molecular concentration of the acetyl-ETG in the organic solvent phase and the concentration of the monomer compounds in the aqueous phase is preferably 1:1 to 10:1.

In this embodiment, PCT is not particularly limited if it is derived from a microorganism having an enzyme that can transfer a CoA group from an acetyl-CoA to 3HP, 3HB or LA but it is Clostridium propionicum, particularly Clostridium propionicum JCM1430 in this embodiment.

Additionally, in this embodiment, PHA synthase is not particularly limited if it is derived from a microorganism having an enzyme that can polymerize 3HB-CoA derivatives, 3HP-CoA derivatives or LA-CoA derivatives, alone, or in combination with each other and synthesize P(3HB), P(3HB-*co-*3HP) or P(3HB-*co-*LA) but it is microorganism-derived that belongs to genus Ralstonia and genus Pseudomonas, particularly, Ralstonia eutropha, and Pseudomonas sp. 61-3. In addition, Ralstonia eutropha ATCC17699-derived and Sequence No. 2 as a preferred PHA synthase in this embodiment.

### [Example]

Next, specific examples in the method for producing a polymer of this embodiment are described.

### [Example 1]

In Example 1, a method for forming acetyl-thioester using acetate as a starting material to produce P(3HB) is described.

### (1) Production of acetyl-thioester

First, using acetate as a raw material, 2 types of acetyl-thioester (acetyl-TP and acetyl-ETG) were prepared [Yuan, W.; Jia, Y.; Tian, J.; Snell, K. D.; Müh, U.; Sinskey, A. J.; Lambalot, R. H.; Walsh, C. T.; Stubbe, J. Arch. Biochem, Biophys. 2001, 394, 87-98.]. Figure 6(1) shows the production process for acetyl-TP and Figure 6(2) shows the production process for acetyl-ETG.

It was confirmed that each substance obtained is esterificated by thin-layer chromatography (TLC) technique, using instrument of Merck Ltd. (Silica Gel F254). The overall structure was found by ¹H-NMR spectrum, using nuclear magnetic resonance (NMR). In NMR measurement, MSL400 spectroscope of Bruker Corporation was employed, with a frequency of 400MHz. All NMR spectra were recorded in deuterated chloroform (CDCl₃) as the solvent, wherein the Figures only show sections of the spectra within a range of 0 to about 6.4 ppm. In the range above 6.4 ppm, there were no relevant signals, therefore this range is omitted and the typical singlet signal of CDCl₃ at 7.24 ppm is not shown.

### (2) PHA synthase (PhaC)

Next, after microbial production of overexpression PhaC was constructed, purified PhaC was obtained (Satoh, Y.; Tajima, K.; Tannai, H.; Munekata, M. J. Biosci. Bioeng. 2003, 95, 335-341).

Firstly, Genomic DNA of Ralstonia eutropha ATCC17699 was treated with restriction enzymes of EcoRI and SmaI (both prepared by TAKARA Bio Inc.). Using pUC 18 (TAKARA Bio Inc.), approx. 5kbp of gene fragment containing a PhaC gene was cloned to obtain a plasmid pTI305.

Next, approx. 1.6kbp of NotI/StuI fragment in a pTI305, a gene fragment having 140bp of BamHI site and SmaI site amplified by PCR according to the following conditions, using pTI305 as a template, and vector pQE 30 (Qiagen) treated with BamHI and SmaI were mixed to be ligated. Then, using this reaction solution, Escherichia coli JM109 was transformed to obtain a plasmid pQEREC having a PhaC gene from a transfectant. By introducing this plasmid to an Escherichia coli BL21, Escherichia coli for preparing PhaC was obtained.

The PCR employed the following primers.
Sense primer: aaggatccatggcgaccggcaaaggcgcgg (Sequence No. 3)
Antisense primer: tgcagcggaccggtggcctcggcc (Sequence No. 4)
The PCR was performed in 30 cycles, each cycle comprising 45-second reaction at 94°C, 30-second reaction at 58°C, and 60-second reaction at 72°C.

Escherichia coli for preparing PhaC obtained was cultured in 1000mL of LB medium containing ampicillin at 30°C for 16 hours. After sonication of the microbial cell bodies accumulated PhaC, soluble protein in the microbial cell body was collected. The collected protein was put in an Ni-NTA agarose gel column (Qiagen) to purify (6×His)-PhaC in one step.

### (3) PCT

Next, using a transformant obtained by introducing a plasmid pCCPP to the Escherichia coli BL21, Clostridium propionicum-derived PCT was produced to obtain a purified PCT by the same approach as the PhaC purification.

The activity of the purified PCT was measured using the monomer-producing reaction and P(3HB) polymerization reaction combined. Specifically, 0.5mL of solution containing 100mM sodium phosphate buffer (pH7.5), 2mM acetyl-CoA and 200mM 3HB, PhaC and PCT was prepared. Then, PCT activity was confirmed by observing the rise in CoA concentration as P (3HB) was produced.

### (4) Production of P(3HB)

Then, P(3HB) was produced, using the acetyl-TP or acetyl-ETG, and the PhaC, and the PCT obtained in the-above-processes. Figure 7(1) shows the reaction process using the acetyl-TP, and Figure 7(2) shows the reaction process using the acetyl-ETG.

Firstly, as an aqueous phase reaction solution, 5mL of solution containing 100mM sodium phosphate buffer (pH7.5), 2.0mM CoA, 10mM (*R*)-3HB and 25U (1mg) PCT was prepared. Next, as an organic solvent phase reaction solution, 5mL of hexane solution containing 10mM acetyl-TP or 10mM acetyl-ETG was prepared. After pouring the aqueous phase reaction solution into a screw cap test tube, the organic solvent phase reaction solution was added thereto. Finally, 5.4U (0.2mg) PhaC was added to the aqueous phase to be reacted at 30°C for 48 hours. The organic solvent phase was removed after the reaction was completed, and 5mL of chloroform was added thereto to extract a product at 70°C for 3 hours. The extract was filtrated with a filter (0.2µm PTFE membrane; Advantec), and 505mL of methanol was added thereto and allowed to stand overnight at 4°C. Afterwards, a produced precipitate was filtrated with a filter (0.2µm PTFE membrane) and collected. After it was vacuum-dried, its yield was measured. 0.2mg of an acetyl-TP product and 2.9mg of an acetyl-ETG product were obtained.

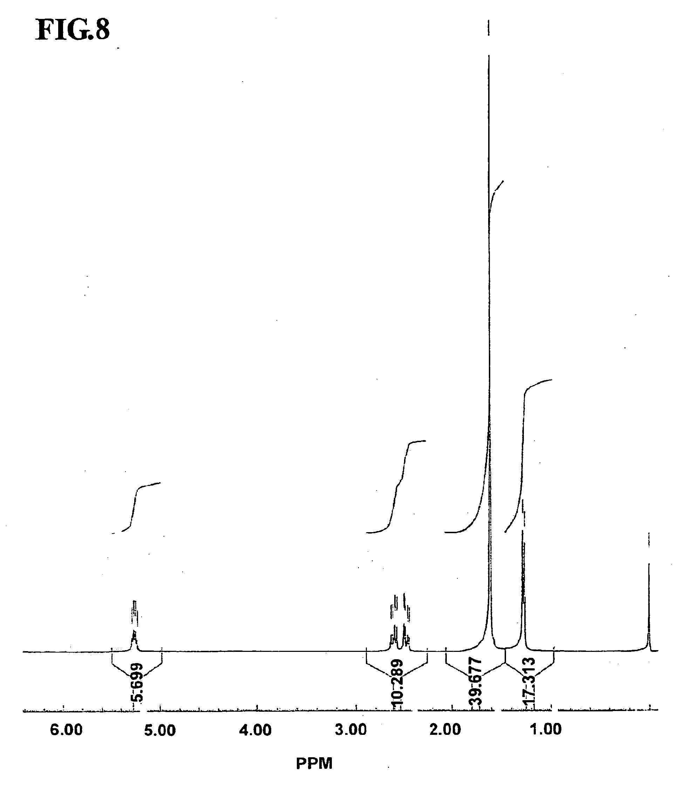

The structure of each product obtained was confirmed using NMR to find out a P(3HB) product. Figure 8 shows ¹H-NMR spectrum using the acetyl-ETG.

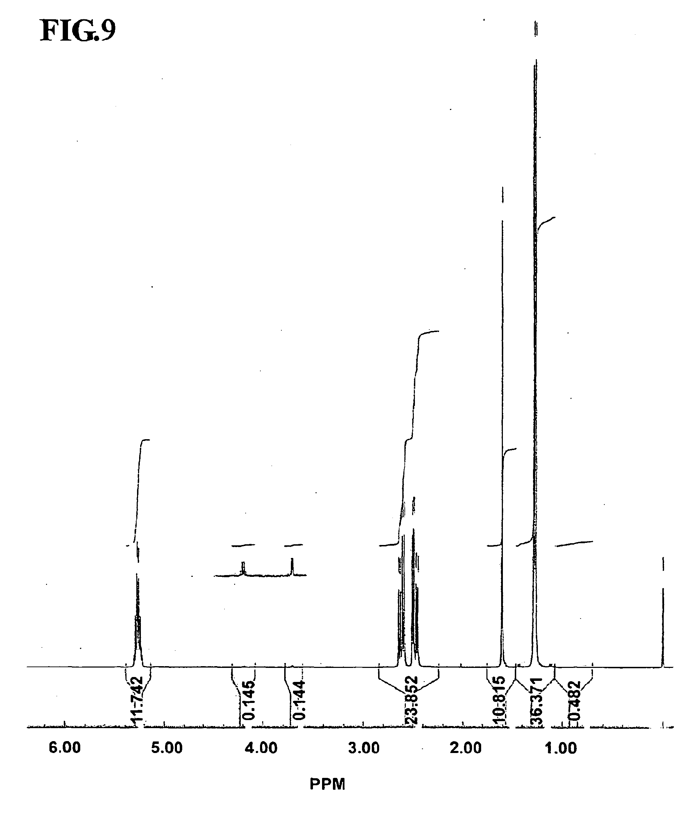

Next, by setting the acetyl-ETG concentration in 500µL of organic solvent phase reaction solution at 1M and the (*R*)-3HB concentration in 5mL of aqueous phase reaction solution at 100mM, P(3HB) was produced under the same conditions to obtain 6.6mg product. The structure of the product obtained was confirmed using NMR measurement to find out a P(3HB) product. Figure 9 shows its ¹H-NMR spectrum.

Then, the molecular weight of the product obtained was measured by gel permeation chromatography (GPC). In GPC measurement method, tandem TSK gel Super HZM-H column (6.0nmLD.×150mm; Tosoh Corporation) was employed and the mobile phase was chloroform with a flow rate of 0.3mL/min. The calibration curve was determined using pure polystyrene. The weight-average molecular weight (Mw) was 8.5×10⁴, and the molecular weight distribution (Mw/Mn) was 1.7.

While the invention in the document WO2004065609 produced a P(3HB) amount of 1.8mg, this invention obtained a P(3HB) amount of 6.6mg, showing approx. 4 times.

From this result, only 2 reaction processes, one process for producing acetyl-thioester from acetate and the other process for synthesizing P (3HB) from acetyl-thioester and released hydroxy acid, can obtain a high-yield ofP (3HB) as a final objective substance.

In addition, in the process for producing acetyl-CoA from acetate, acetyl-ETG from ETG can be used to form acetyl-CoA, rather than acetyl-TP from conventional highly toxic TP. Without using a purified enzyme or expensive ATP, acetyl-CoA can be prepared from a low-cost acetate, thereby providing significant advantages in industrial application.

### [Example 2]

In Example 2, P(3HB) polymerization reaction rate and production amount were discussed according to the type of acetyl-thioester.

As acetyl-thioester, acetyl-TP and acetyl-ETG were employed. In P(3HB) production process, P(3HB) polymerization reaction causes precipitate and makes the reaction solution cloudy. As the polymerization reaction is completed, the reaction solution becomes transparent, with white precipitates. Thus, the progress of P(3HB) production can be found from a visual observation and the turbidity in the reaction solution. The progress of P(3HB) production was measured from the turbidity in the reaction solution using an absorptiometer.

Specifically, 1.55mL of an organic solvent phase reaction solution, a hexane solution containing 10mM of acetyl-TP or acetyl-ETG was added to 1.55mL of an aqueous phase reaction solution containing 100mM sodium phosphate buffer (pH7.5), 10mM (*R*)-3HB, 2.0mM CoA and 7.5U (0.3mg) PCT. Finally, 1.6U (0.06mg) PhaC was added to the aqueous phase reaction solution to cause a reaction at 30°C and the turbidity of the reaction solution was measured at a wavelength of 600nm using an absorptiometer (Hitachi High-Technologies Corporation).

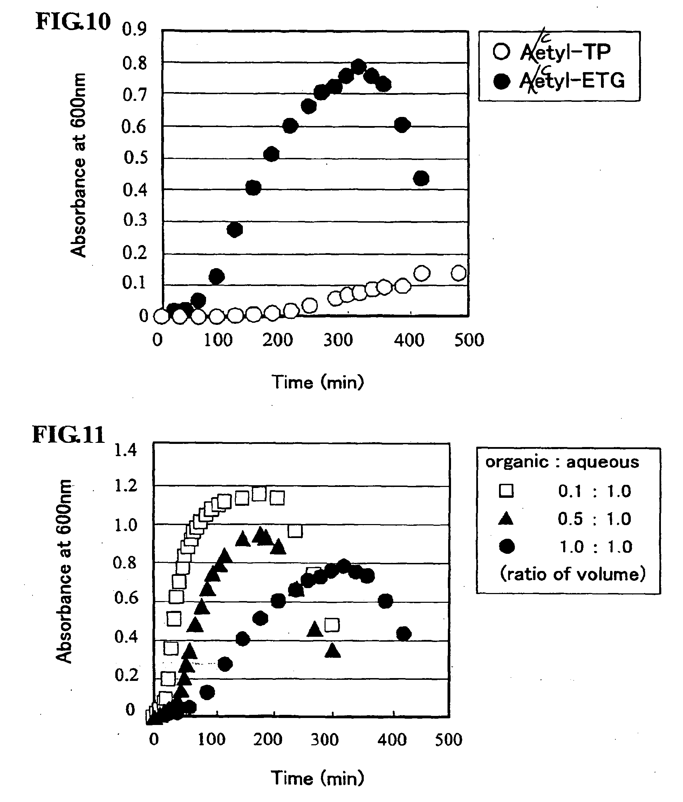

As a result, as shown in Figure 10, the absorbance of the reaction solution increased in the acetyl-ETG (indicated as • in Figure 10), then decreased after it reached 0.78 in 320 minutes. According to a visual observation, the solution started to become clouded 60 minutes later and in 120 minutes white precipitates were found. Meanwhile, no peak was found in the acetyl-TP (indicated as o in Figure 10) even 500 minutes later, with an absorbance of the reaction solution at 0.2 or less. Therefore, it was found that the acetyl-ETG provides more rapid reaction and higher-yield product than the acetyl-TP.

It is known that TP is highly toxic due to its blocking activity for PhaC. According to this Example, as opposed to the acetyl-TP, the acetyl-ETG is produced without using TP and also can synthesize P(3HB) suitable for industrial application in an immediate and high-yield manner.

### [Example 3]

In Example 3, by changing the ratio of the concentration of the acetyl-ETG in the organic solvent phase and the concentration of (*R*)-3HB in the aqueous phase, the synthetic reaction rate for P(3HB) was discussed.

Firstly, a hexane solution containing 0.5mmol acetyl-ETG was prepared as an organic solvent phase reaction solution, and 1.55mL of solution containing 0.5mmol (*R*)-3HB, 100mM sodium phosphate buffer (pH7.5), 2.0mM CoA and 7.5U (0.3mg) PCT was prepared as an aqueous phase reaction solution. By maintaining the acetyl-ETG amount in this organic solvent phase reaction solution and the (R)-3HB amount in the aqueous phase reaction solution at constant levels, the amount of the organic solvent phase reaction solution was changed. Specifically, the ratio of the volume of organic solvent phase reaction solution and the volume of the aqueous phase reaction solution was determined at 0.1:1 (indicated as □ in Figure 11), 0.5:1 (indicated as ▲ in Figure 11) and 1:1 (indicated as ● in Figure 11). Then, 1.6U (0.06mg) PhaC was added to the aqueous phase to be reacted at 30°C. The turbidity of the reaction solutions in each system was measured at a wavelength of 600nm with an absorptiometer.

As a result, as shown in Figure 11, it was found that the time for reaching the peak in the absorbance and its absorbance are long and low, respectively, as the volumetric ratio of the organic solvent phase grows. In the system in which the ratio of the organic solvent phase and the aqueous phase is 0.1:1 (indicated as □ in Figure 11), the solution started to become clouded 10 minutes after the reaction started and 60 minutes later, the absorbance reached the peak. Compared with other systems, the time for reaching the peak in the absorbance is the shortest and the absorbance was highest.

From these results, P(3HB) can be synthesized in an immediate and high-yield manner in favorable industrial application when the volumetric ratio of the organic solvent phase and the aqueous phase is 1:1 to 0.1:1, or the ratio (mmol/L) of the concentration of acetyl-ETG and the concentration of (*R*)-3HB is 1:1 to 10:1, and most preferably when the ratio of the concentration in mol is 10:1.

### [Example 4]

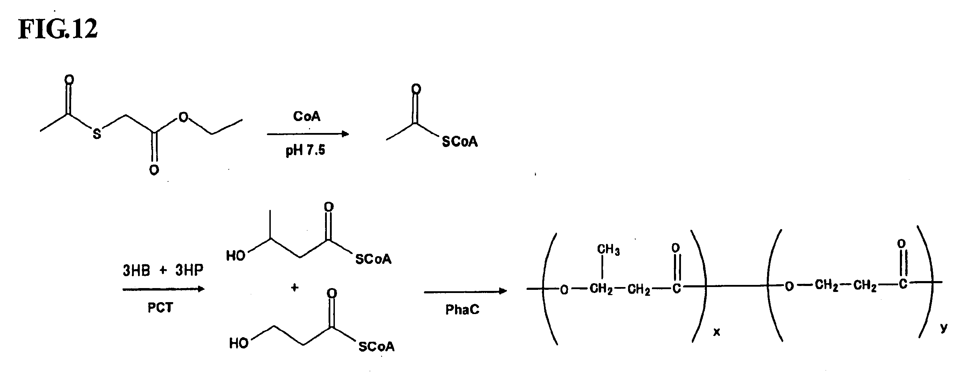

In Example 4, using acetyl-ETG, PhaC and PCT obtained in Examples 1(1) to (3), P(3HB-co-3HP) was produced. Figure 12 shows the reaction process.

Firstly, as an aqueous phase reaction solution, 5mL of a solution containing 100mM sodium phosphate buffer (pH7.5), and 2.0mM CoA, and 50mM 3HP or 50mM *(R*)-3HB, and 4.3U (2.5mg) PCT was prepared. Next, as an organic solvent phase reaction solution, 500µL of hexane solution containing 1.0M acetyl-ETG was prepared. Then, after pouring the aqueous phase reaction solution into a screw cap test tube, the organic solvent phase reaction solution was added thereto. Finally, 5U (2.5mg) PhaC was added to the aqueous phase to be reacted at 30°C for 24 hours. After the reaction was completed, the organic solvent phase was removed and 5mL of chloroform was added thereto, and a product was extracted at 70°C for 3 hours. The extract was filtrated with a filter (0.2 µm PTFE membrane; Advantec) and 50mL methanol was added thereto and allowed to stand overnight at 4°C. The resulting precipitate was filtrated with a filter (0.2µm PTFE membrane) and collected. Vacuum-dried yield was measured to obtain 3.2mg of product.

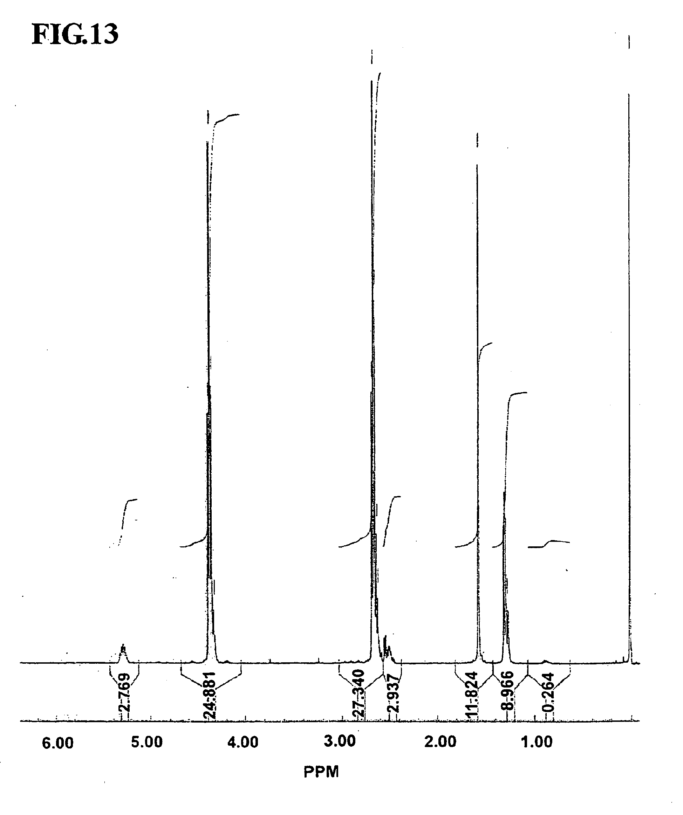

The structure of each product obtained was confirmed in NMR to find out a P(3HB-*co-*3HP) product. Figure 13 shows its ¹H-NMR spectrum.

From these results, the method for producing a polymer of this embodiment can produce not only P(3HB), but also P(3HB-*co-*3HP).

### [Example 5]

In Example 5, using acetyl-ETG and PCT obtained in Examples 1(1) to (3) and PhaC obtained as follows, P(3HB-*co-*LA) was produced. Figure 14 shows the reaction process.

### (1) PhaC

After microbial production of overexpression PhaC was constructed, purified PhaC was obtained (Satoh, Y.; Tajima, K.; Tannai, H.; Munekata, M. J. Biosci. Bioeng. 2003, 95, 335-341 ).

Firstly, Gene fragment (Sequence No. 1) which codes for Sequence No. 2, the amino-acid sequence of Pseudomonas sp. 61-3-derived PhaC that was disclosed in the document (WO2003-100055), was chemically-synthesized. This gene fragment was inserted into pUC19 treated with restriction enzymes of SacI (TAKARA Bio Inc.) to obtain a plasmid pUC1dm.

Next, a gene fragment having 1.6kbp of BamHI site and HindIII site amplified by PCR according to the following conditions, using pUC1 dm as a template, and vector pQE 30 (Qiagen) treated with BamHI and HindIII were mixed to be ligated. Then, using this reaction solution, Escherichia coli JM109 was transformed to obtain a plasmid pQC1 dm having the PhaC gene from the transfectant. By transfecting this plasmid to an Escherichia coli BL21, Escherichia coli for preparing PhaC was obtained.

The PCR employed the following primers.
Sense primer: ccggatccagtaacaagaatagcgatgacttga (Sequence No. 5)
Antisense primer: tttaagcttaacgttcatgcacatacgtg (Sequence No. 6)
The PCR was performed in 30 cycles, each cycle comprising 60-second reaction at 94°C, 30-second reaction at 55°C, and 100-second reaction at 72°C.

Escherichia coli for preparing PhaC obtained was cultured in 1000mL of LB medium containing ampicillin at 30°C for 3 hours and more cultured in the isopropyl-β-D-thiogalactopyranoside (IPTG ; 0.25M final concentration) -added LB medium at 30°C for 16 hours. After sonication of the microbial cell bodies accumulated PhaC, soluble protein in the microbial cell body was collected. The collected protein was put in an Ni-NTA agarose gel column (Qiagen) to purify (6×this)-PhaC in one step.

### (2) Production of P (3HB-co-LA)

Firstly, as an aqueous phase reaction solution, 5mL of solution containing 100mM sodium phosphate buffer (pH7.5), 1.0mM CoA, 50mM LA (D-enantiomer of LA), 50mM (*R*)-3HB and 50U (2.5mg) PCT was prepared. Next, as an organic solvent phase reaction solution, 5mL of hexane solution containing 100mM acetyl-ETG was prepared. After pouring the aqueous phase reaction solution into a screw cap test tube, the organic solvent phase reaction solution was added thereto. Finally, 0.05U (2.5mg) PhaC was added to the aqueous phase to be reacted at 30°C for 72 hours. The organic solvent phase was removed after the reaction was completed, and 5mL of chloroform was added thereto to extract a product at 70°C for 3 hours. The extract was filtrated with a filter (0.2µm PTFE membrane; Advantec), and 50mL of methanol was added thereto and allowed to stand overnight at 4°C. Afterwards, a produced precipitate was filtrated with a filter (0.2µm PTFE membrane) and collected. After it was vacuum-dried, its yield was measured. 0.1 mg of a product was obtained.

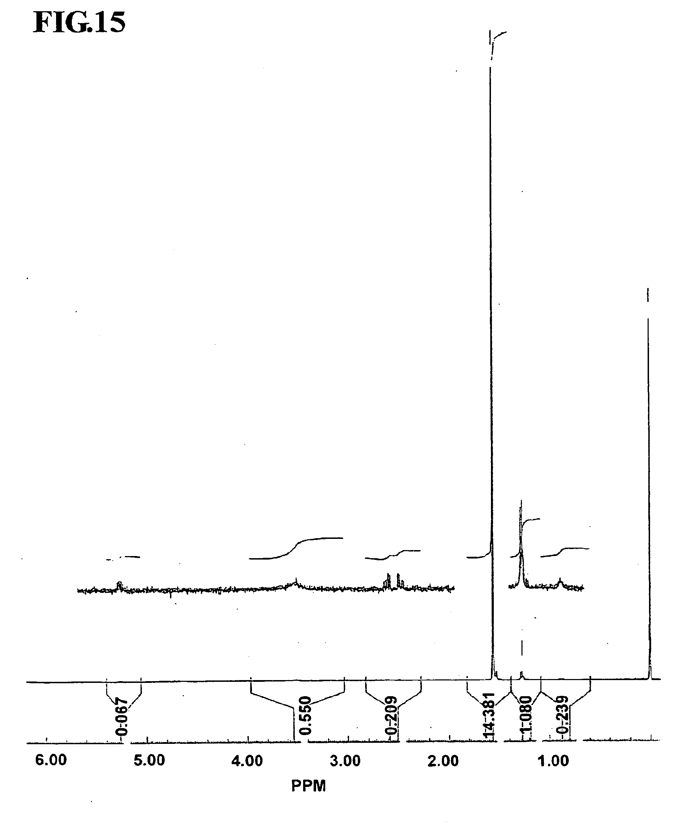

The structure of the product obtained was confirmed using NMR to find out a P(3HB-*co*-LA) product. Figure 15 shows this ¹H-NMR spectrum.

From this results, the method for producing a polymer of this embodiment can produce not only P(3HB) or P(3HB-*co-*3HP), but also P (3HB-*co-*LA).

### [Example 6]

In Example 6, PCT substrate specificity was discussed, in order to examine whether the method for producing a polymer in the aqueous -organic solvent two-phase systems performed in the preceding Examples can be applied even in hydroxy acid or unsaturated fatty acid other than (*R*)-3HB and 3HP. Clostridium propionicum-derived PCT obtained in Example 1 (3) was used.

In the method in Example 1 (3), various types of hydroxy acid or unsaturated fatty acid were added, instead of (*R*)-3HB, to be reacted for 24 hours with no PhaC added. The compound obtained was analyzed by HPLC (Shimazu). The HPLC measurement employed Mightysil RP-18 GP Aqua-column (4.6nm I.D.×150mm; Kanto Chemical), and the mobile phase was liquid A (50mM NaH₂PO₄ solution containing 10wt% methanol) or liquid B ( 50mM NaH₂PO₄ solution containing 40wt% methanol). The proportion of the liquid B is 0% (0 to 5 minutes), 0 to 20% (5 to 10 minutes), 20 to 100% (15 to 17.5 minutes), 100% (17.5 to 22.5 minutes), 100 to 0% (22.5 to 25 minutes) or 0% (25 to 30 minutes). The flow rate was 0.7mL/min, and the detector was an ultraviolet absorptiometer.

As a result, as PCT substrate, or hydroxy acid or unsaturated fatty acid as monomer component, the method can be applied in LA, 3HP, 3HB, 4HB, crotonate, pentenate, serine, glycolate and acrylate.

Clostridium propionicum-derived PCT was used in the Examples, but when other strain-derived PCT is employed, it seems that the method according to the present invention can be applied in hydroxy acid not shown in the Examples. Specifically, by selecting hydroxy acid, or strain of microoganism from which PCT and PHA synthase derives, accordingly, a polymer having a desired monomer composition can be produced.

From the above observations of this embodiment, biodegradable polymers can be produced with the following characteristics, according to the extremely industrially favorable production method.
1. Compared with conventional synthetic methods, this method can produce a polymer in an immediate and high-yield manner.
2. In fact, only two easy reaction processes, one process for forming an acetyl-thioester from acetate and the other process for synthesizing a polymer from acetyl-thioester and released hydroxy acid, can obtain a polymer.
3. An acetyl-CoA can be formed from a low-cost acetate, without using purified enzyme, expensive ATP or highly toxic TP to produce polymers in an immediate and high-yield manner.
4. With wider varieties of monomer components to be selected, a polymer can be produced so as to be provided with a desired composition.

## Claims

1. A method for producing a polyester polymer comprising units selected from serine and/or hydroxyalkanoate(s), wherein said method comprises the following reactions:
a) a chemical thioester exchange reaction, wherein an acetyl-thioester is reacted with CoA for forming acetyl-CoA ;
b) a monomer-producing reaction, wherein at least one monomer precursor compound is reacted with said acetyl-CoA for forming a (monomer precursor)-CoA derivative; and
c) a polymerization reaction, wherein said (monomer precursor)-CoA derivative is polymerized for forming said polyester polymer comprising units of said monomer.

2. The method for producing a polyester polymer according to Claim 1, wherein the hydroxyalkanoate units are selected from lactate (LA), 3-hydroxypropionate (3HP), 3-hydroxybutyrate (3HB), or 4-hydroxybutyrate (4HB), alone or in combination with each other or with other units.

3. The method for producing a polyester polymer according to Claim 2, wherein said polyester is polylactate (PLA), poly-3-hydroxypropionate {P(3HP)}, poly-3-hydroxybutyrate {P(3HB)}, poly-4-hydroxybutyrate {P(4HB)}, LA-3HP-copolyester {P(LA-*co-*3HP)}, LA-3HB-copolyester {P(LA-*co-*3HB)}, LA-4HB-copolyester {P(LA-*co-*4HB)}, 3HP-3HB-copolyester {P(3HP-*co-*3HB)}, 3HP-4HB-copolyester {P(3HP-*co-*4HB)}, or 3HB-4HB-copolyester {P(3HB-*co-*4HB)}.

4. The method for producing a polyester polymer according to Claim 2 or 3, wherein said lactate (LA) is Dextro-rotatory-lactate (D-lactate).

5. The method for producing a polyester polymer according to any one of Claims 1 to 4, wherein the chemical thioester exchange reaction comprises forming acetyl-CoA from CoA, which is released from the (monomer precursor)-CoA derivative in said polymerization reaction, and said acetyl-thioester.

6. The method for producing a polyester polymer according to any one of Claims 1 to 5, wherein said acetyl-thioester of the chemical thioester exchange reaction is prepared from an acetate and a thiol compound by a thioesterification reaction.

7. The method for producing a polyester polymer according to any one of Claims 1 to 6, wherein said thioesterification reaction comprises forming acetyl-thioester from acetate, which is released in said monomer-producing reaction, and thiol compounds.

8. The method for producing a polyester polymer according to Claim 6 or 7, wherein the thiol compound is ethylthioglycolate (ETG).

9. The method for producing a polyester polymer according to any one of Claims 1 to 8, wherein an enzyme used for the monomer-producing reaction is one using acetyl-CoA as a substrate.

10. The method for producing a polyester polymer according to any one of Claims 1 to 9, wherein the chemical thioester exchange reaction, the monomer-producing reaction and the polymerization reaction proceed concurrently in an one pot reaction system, and wherein the one pot reaction system comprises an organic solvent phase and an aqueous solvent phase.

11. The method for producing a polyester polymer according to Claim 10, wherein the organic solvent phase contains the acetyl-thioester, and wherein the aqueous phase contains CoA, the monomer precursor compound and two enzymes catalyzing said reaction (b) and (c), respectively.

12. The method for producing a polyester polymer according to Claim 10 or 11, wherein a ratio of the concentration of the acetyl-thioester and the concentration of the monomer precursor compound is 1:1 to 10:1.

13. Use of a method according to any one of Claims 1 to 12 for preparing a polyester polymer comprising units selected from serine and/or hydroxyalkanoate(s), wherein said polyester polymer has a molecular weight distribution M_{w}/Mₙ of between 1 and 2.5.

## Patentansprüche

1. Verfahren zum Herstellen eines Polyesterpolymers, das Einheiten ausgewählt aus Serin und/oder Hydroxyalkanoat(en) umfasst, wobei das Verfahren die folgenden Reaktionen umfasst:
a) eine chemische Thioester-Austauschreaktion, wobei ein Acetyl-Thioester mit CoA zum Bilden von Acetyl-CoA reagiert wird;
b) eine Monomer erzeugende Reaktion, wobei mindestens eine Monomerprecursorverbindung mit dem Acetyl-CoA zum Bilden eines (Monomerprecursor)-CoA-Derivats reagiert wird; und
c) eine Polymerisierungsreaktion, wobei das (Monomerprecursor)-CoA-Derivat zum Bilden des Polyesterpolymers, das Einheiten des Monomers umfasst, polymerisiert wird.

2. Verfahren zum Herstellen eines Polyesterpolymers gemäß Anspruch 1, wobei die Hydroxyalkanoat-Einheiten aus Lactat (LA), 3-Hydroxypropionat (3HP), 3-Hydroxybutyrat (3HB) oder 4-Hydroxybutyrat (4HB), alleine oder in Kombination miteinander oder mit anderen Einheiten, ausgewählt werden.

3. Verfahren zum Herstellen eines Polyesterpolymers gemäß Anspruch 2, wobei der Polyester Polylactat (PLA), Poly-3-hydroxypropionat {P(3HP)}, Poly-3-hydroxybutyrat {P(3HB)}, Poly-4-hydroxybutyrat {P(4HB)}, LA-3HP-Copolyester {P(LA-*co-*3HP)}, LA-3HB-Copolyester {P(LA-*co-*3HB)}, LA-4HB-Copolyester {P(LA-*co-*4HB)}, 3HP-3HB-Copolyester {P(3HP-*co-*3HB)}, 3HP-4HB-Copolyester {P(3HP-*co-*4HB)} oder 3HB-4HB-Copolyester {P(3HB-*co-*4HB)} ist.

4. Verfahren zum Herstellen eines Polyesterpolymers gemäß Anspruch 2 oder 3, wobei das Lactat (LA) rechtsdrehendes Lactat (D-Lactat) ist.

5. Verfahren zum Herstellen eines Polyesterpolymers gemäß irgendeinem der Ansprüche 1 bis 4, wobei die chemische Thioester-Austauschreaktion das Bilden von Acetyl-CoA aus CoA, welches aus dem (Monomerprecursor)-CoA-Derivat bei der Polymerisationsreaktion freigesetzt wird, und dem Acetyl-Thioester umfasst.

6. Verfahren zum Herstellen eines Polyesterpolymers gemäß irgendeinem der Ansprüche 1 bis 5, wobei der Acetyl-Thioester der chemischen Thioester-Austauschreaktion aus einem Acetat und einer Thiolverbindung durch eine Thioveresterungsreaktion hergestellt wird.

7. Verfahren zum Herstellen eines Polyesterpolymers gemäß irgendeinem der Ansprüche 1 bis 6, wobei die Thioveresterungsreaktion das Bilden von Acetyl-Thioester aus Acetat, welches in der Monomer-erzeugenden Reaktion freigesetzt wird, und Thiolverbindungen umfasst.

8. Verfahren zum Herstellen eines Polyesterpolymers gemäß Anspruch 6 oder 7, wobei die Thiolverbindung Ethylthioglykolat (ETG) ist.

9. Verfahren zum Herstellen eines Polyesterpolymers gemäß irgendeinem der Ansprüche 1 bis 8, wobei ein für die Monomer erzeugende Reaktion verwendetes Enzym eines ist, das Acetyl-CoA als Substrat verwendet.

10. Verfahren zum Herstellen eines Polyesterpolymers gemäß irgendeinem der Ansprüche 1 bis 9, wobei die chemische Thioester-Austauschreaktion, die Monomer erzeugende Reaktion und die Polymerisierungsreaktion gleichzeitig in einem Eintopf-Reaktionssystem ablaufen, und wobei das Eintopf-Reaktions-System eine organische Lösungsmittelphase und eine wässrige Lösungsmittelphase umfasst.

11. Verfahren zum Herstellen eines Polyesterpolymers gemäß Anspruch 10, wobei die organische Lösungsmittelphase den Acetyl-Thioester enthält, und wobei die wässrige Phase CoA, die Monomerprecursorverbindung und zwei jeweils die Reaktion (b) and (c) katalysierende Enzyme enthält.

12. Verfahren zum Herstellen eines Polyesterpolymers gemäß Anspruch 10 oder 11, wobei ein Verhältnis von der Konzentration des Acetyl-Thioesters zur Konzentration der Monomerprecursorverbindung 1 : 1 bis 10 : 1 ist.

13. Verwendung eines Verfahrens gemäß irgendeinem der Ansprüche 1 bis 12 zum Herstellen eines Polyesterpolymers, das Einheiten ausgewählt aus Serin und/oder Hydroxyalkanoat(en) umfasst, wobei das Polyesterpolymer eine Molekulargewichtsverteilung M_{w}/Mₙ zwischen 1 und 2,5 aufweist.

## Revendications

1. Procédé de préparation d'un polymère de polyester comprenant des motifs choisis parmi la sérine et/ou le(s) hydroxyalcanoate(s), dans lequel ledit procédé comprend les réactions suivantes :
a) une réaction d'échange chimique de thioester, dans laquelle un acétyl-thioester est mis à réagir avec du CoA pour former de l'acétyl-CoA ;
b) une réaction de production d'un monomère, dans laquelle au moins un composé précurseur monomère est mis à réagir avec ledit acétyl-CoA pour former un dérivé de (précurseur monomère)-CoA ; et
c) une réaction de polymérisation, dans laquelle ledit dérivé de (précurseur monomère)-CoA est polymérisé pour former ledit polymère de polyester comprenant les motifs dudit monomère.

2. Procédé de production d'un polymère de polyester selon la revendication 1, dans lequel les motifs hydroxyalcanoates sont choisis parmi le lactate (LA), le 3-hydroxypropionate (3HP), le 3-hydroxybutyrate (3HB) ou le 4-hydroxybutyrate (4HB), seuls ou en combinaison les uns avec les autres ou avec d'autres motifs.

3. Procédé de production d'un polymère de polyester selon la revendication 2, dans lequel ledit polyester est un polylactate (PLA), un poly-3-hydroxypropionate {P(3HP)}, un poly-3-hydroxybutyrate {P(3HB)}, un poly-4-hydroxybutyrate {P(4HB)}, un LA-3HP-copolyester {P(LA-co-3HP)}, un LA-3HB-copolyester {P(LA-co-3HB)}, un LA-4HB-copolyester {P(LA-co-4HB)}, un 3HP-3HB-copolyester {P(3HP-co-3HB)}, un 3HP-4HB-copolyester {P(3HP-co-4HB)}, ou un 3HB-4HB-copolyester {P(3HB-co-4HB)}.

4. Procédé de production d'un polymère de polyester selon la revendication 2 ou 3, dans lequel ledit lactate (LA) est le lactate dextrogyre (D-lactate).

5. Procédé de production d'un polymère de polyester selon l'une quelconque des revendications 1 à 4, dans lequel la réaction d'échange chimique de thioester comprend la formation d'acétyl-CoA à partir de CoA, qui est libéré du dérivé de (précurseur monomère)-CoA dans ladite réaction de polymérisation, et dudit acétyl-thioester.

6. Procédé de production d'un polymère de polyester selon l'une quelconque des revendications 1 à 5, dans lequel ledit acétyl-thioester de la réaction d'échange chimique de thioester est préparé à partir d'un acétate et d'un composé thiol par une réaction de thioestérification.

7. Procédé de production d'un polymère de polyester selon l'une quelconque des revendications 1 à 6, dans lequel ladite réaction de thioestérification comprend la formation d'acétyl-thioester à partir d'acétate, qui est libéré dans ladite réaction de production de monomère, et de composés thiols.

8. Procédé de production d'un polymère de polyester selon la revendication 6 ou 7, dans lequel le composé thiol est le thioglycolate d'éthyle (ETG).

9. Procédé de production d'un polymère de polyester selon l'une quelconque des revendications 1 à 8, dans lequel une enzyme utilisée pour la réaction de production de monomère est une enzyme utilisant l'acétyl-CoA comme substrat.

10. Procédé de production d'un polymère de polyester selon l'une quelconque des revendications 1 à 9, dans lequel la réaction d'échange chimique de thioester, la réaction de production d'un monomère et la réaction de polymérisation sont réalisées de manière simultanée dans un système réactionnel utilisant un unique récipient, et dans lequel le système réactionnel utilisant un unique récipient comprend une phase à base de solvant organique et une phase à base de solvant aqueux.

11. Procédé de production d'un polymère de polyester selon la revendication 10, dans lequel la phase à base de solvant organique comprend l'acétyl-thioester, et dans lequel la phase aqueuse comprend le CoA, le composé précurseur monomère et deux enzymes catalysant lesdites réactions (b) et (c) respectivement.

12. Procédé de production d'un polymère de polyester selon la revendication 10 ou 11, dans lequel un rapport de la concentration de l'acétyl-thioester et de la concentration du composé précurseur monomère est compris dans la plage allant de 1:1 à 10:1.

13. Utilisation d'un procédé selon l'une quelconque des revendications 1 à 12, pour la préparation d'un polymère de polyester comprenant des motifs choisis parmi la sérine et/ou le(s) hydroxyalcanoate(s), dans laquelle ledit polymère de polyester possède une distribution de poids moléculaire Mₘ/Mₙ comprise entre 1 et 2,5.


### SEQUENCE LISTING

<110> AgriBioIndustry Inc.
   National University Corporation Hokkaido university
<120> Method for producing polymer
<130> 2007-92-J2.
<160> 6
<170> PatentIn version 3.3
<210> 1
   <211> 1680
   <212> DNA
   <213> Pseudomonas sp.61-3
<400> 1
   atgagtaaca agaatagcga tgacttgaat cgtcaagcct cggaaaacac cttggggctt     60
   aaccctgtca tcggcctgcg tggaaaagat ctgctgactt ctgcccgaat ggttttaacc    120
   caagccatca aacaacccat tcacagcgtc aagcacgtcg cgcattttgg catcgagctg    180
   aagaacgtga tgtttggcaa atcgaagctg caaccggaaa gcgatgaccg tcgtttcaac    240
   gaccccgcct ggagtcagaa cccactctac aaacgttatc tacaaaccta cctggcgtgg    300
   cgcaaggaac tccacgactg gatcggcaac agcaaactgt ccgaacagga catcaatcgc    360
   gctcacttcg tgatcaccct gatgaccgaa gccatggccc cgaccaacag tgcggccaat    420
   ccggcggcgg tcaaacgctt cttcgaaacc ggcggtaaaa gcctgctcga cggcctcaca    480
   catctggcca aggacctggt aaacaacggc ggcatgccga gccaggtgga catgggcgct    540
   ttcgaagtcg gcaagagtct ggggacgact gaaggtgcag tggttttccg caacgacgtc    600
   ctcgaattga tccagtaccg gccgaccacc gaacaggtgc atgagcgacc gctgctggtg    660
   gtcccaccgc agatcaacaa gttttatgtg tttgacctga gcccggataa aagcctggcg 720
   cgcttctgcc tgagcaacaa ccagcaaacc tttatcgtca gctggcgcaa cccgaccaag 780
   gcccagcgtg agtggggtct gtcgacttac atcgatgcgc tcaaagaagc cgtcgacgta 840
   gtttccgcca tcaccggcag caaagacatc aacatgctcg gcgcctgctc cggtggcatt 900
   acctgcaccg cgctgctggg tcactacgcc gctctcggcg agaagaaggt caatgccctg 960
   acccttttgg tcaccgtgct cgacaccacc ctcgactccc aggttgcact gttcgtcgat 1020
   gagaaaaccc tggaagctgc caagcgtcac tcgtatcagg ccggcgtgct ggaaggccgc 1080
   gacatggcca aagtcttcgc ctggatgcgc cctaacgacc tgatctggaa ctactgggtc 1140
   aacaactacc tgctgggtaa cgagccaccg gtcttcgaca ttcttttctg gaacaacgac 1200
   accacccggt tgcctgctgc gttccacggc gatctgatcg aaatgttcaa aaataaccca 1260
   ctggtgcgcg ccaatgcact cgaagtgagc ggcacgccga tcgacctcaa acaggtcact 1320
   gccgacatct actccctggc cggcaccaac gatcacatca cgccctggaa gtcttgctac 1380
   aagtcggcgc aactgttcgg tggcaaggtc gaattcgtgc tgtccagcag tgggcatatc 1440
   aagagcattc tgaacccgcc gggcaatccg aaatcacgtt acatgaccag caccgacatg 1500
   ccagccaccg ccaacgagtg gcaagaaaac tcaaccaagc acaccgactc ctggtggctg 1560
   cactggcagg cctggcaggc cgagcgctcg ggcaaactga aaaagtcccc gaccagcctg 1620
   ggcaacaagg cctatccgtc aggagaagcc gcgccgggca cgtatgtgca tgaacgttaa 1680
<210> 2
   <211> 559
   <212> PRT
   <213> Pseudomonas sp.61-3
<400> 2
   Met Ser Asn Lys Asn Ser Asp Asp Leu Asn Arg Gln Ala Ser Glu Asn
   1 5 10 15
   Thr Leu Gly Leu Asn Pro Val Ile Gly Leu Arg Gly Lys Asp Leu Leu
   20 25 30
   Thr Ser Ala Arg Met Val Leu Thr Gln Ala Ile Lys Gln Pro Ile His
   35 40 45
   Ser Val Lys His Val Ala His Phe Gly Ile Glu Leu Lys Asn Val Met
   50 55 60
   Phe Gly Lys Ser Lys Leu Gln Pro Glu Ser Asp Asp Arg Arg Phe Asn
   65 70 75 80
   Asp Pro Ala Trp Ser Gln Asn Pro Leu Tyr Lys Arg Tyr Leu Gln Thr
   85 90 95
   Tyr Leu Ala Trp Arg Lys Glu Leu His Asp Trp Ile Gly Asn Ser Lys
   100 105 110
   Leu Ser Glu Gln Asp Ile Asn Arg Ala His Phe Val Ile Thr Leu Met
   115 120 125
   Thr Glu Ala Met Ala Pro Thr Asn Ser Ala Ala Asn Pro Ala Ala Val
   130 135 140
   Lys Arg Phe Phe Glu Thr Gly Gly Lys Ser Leu Leu Asp Gly Leu Thr
   145 150 155 160
   His Leu Ala Lys Asp Leu Val Asn Asn Gly Gly Met Pro Ser Gln Val
   165 170 175
   Asp Met Gly Ala Phe Glu Val Gly Lys Ser Leu Gly Thr Thr Glu Gly
   180 185 190
   Ala Val Val Phe Arg Asn Asp Val Leu Glu Leu Ile Gln Tyr Arg Pro
   195 200 205
   Thr Thr Glu Gln Val His Glu Arg Pro Leu Leu Val Val Pro Pro Gln
   210 215 220
   Ile Asn Lys Phe Tyr Val Phe Asp Leu Ser Pro Asp Lys Ser Leu Ala
   225 230 235 240
   Arg Phe Cys Leu Ser Asn Asn Gln Gln Thr Phe Ile Val Ser Trp Arg
   245 250 255
   Asn Pro Thr Lys Ala Gln Arg Glu Trp Gly Leu Ser Thr Tyr Ile Asp
   260 265 270
   Ala Leu Lys Glu Ala Val Asp Val Val Ser Ala Ile Thr Gly Ser Lys
   275 280 285
   Asp Ile Asn Met Leu Gly Ala Cys Ser Gly Gly Ile Thr Cys Thr Ala
   290 295 300
   Leu Leu Gly His Tyr Ala Ala Leu Gly Glu Lys Lys Val Asn Ala Leu
   305 310 315 320
   Thr Leu Leu Val Thr Val Leu Asp Thr Thr Leu Asp Ser Gln Val Ala
   325 330 335
   Leu Phe Val Asp Glu Lys Thr Leu Glu Ala Ala Lys Arg His Ser Tyr
   340 345 350
   Gln Ala Gly Val Leu Glu Gly Arg Asp Met Ala Lys Val Phe Ala Trp
   355 360 365
   Met Arg Pro Asn Asp Leu Ile Trp Asn Tyr Trp Val Asn Asn Tyr Leu
   370 375 380
   Leu Gly Asn Glu Pro Pro Val Phe Asp Ile Leu Phe Trp Asn Asn Asp
   385 390 395 400
   Thr Thr Arg Leu Pro Ala Ala Phe His Gly Asp Leu Ile Glu Met Phe
   405 410 415
   Lys Asn Asn Pro Leu Val Arg Ala Asn Ala Leu Glu Val Ser Gly Thr
   420 425 430
   Pro Ile Asp Leu Lys Gln Val Thr Ala Asp Ile Tyr Ser Leu Ala Gly
   435 440 445
   Thr Asn Asp His Ile Thr Pro Trp Lys Ser Cys Tyr Lys Ser Ala Gln
   450 455 460
   Leu Phe Gly Gly Lys Val Glu Phe Val Leu Ser Ser Ser Gly His Ile
   465 470 475 480
   Lys Ser Ile Leu Asn Pro Pro Gly Asn Pro Lys Ser Arg Tyr Met Thr
   485 490 495
   Ser Thr Asp Met Pro Ala Thr Ala Asn Glu Trp Gln Glu Asn Ser Thr
   500 505 510
   Lys His Thr Asp Ser Trp Trp Leu His Trp Gln Ala Trp Gln Ala Glu
   515 520 525
   Arg Ser Gly Lys Leu Lys Lys Ser Pro Thr Ser Leu Gly Asn Lys Ala
   530 535 540
   Tyr Pro Ser Gly Glu Ala Ala Pro Gly Thr Tyr Val His Glu Arg
   545 550 555
<210> 3
   <211> 30
   <212> DNA
   <213> Artificial sequence
<220>
   <223> sense primer
<400> 3
   aaggatccat ggcgaccggc aaaggcgcgg 30
<210> 4
   <211> 24
   <212> DNA
   <213> Artificial sequence
<220>
   <223> antisense primer
<400> 4
   tgcagcggac cggtggcctc ggcc 24
<210> 5
   <211> 33
   <212> DNA
   <213> Artificial Sequence
<220>
   <223> sense primer
<400> 5
   ccggatccag taacaagaat agcgatgact tga 33
<210> 6
   <211> 29
   <212> DNA
   <213> Artificial Sequence
<220>
   <223> antisense primer
<400> 6
   tttaagctta acgttcatgc acatacgtg 29